# EUROPEAN PATENT APPLICATION

(11) **EP 3 831 886 A1**
(43) Date of publication of application: **09.06.2021**
(21) Application number: 19845064.5
(22) Date of filing: 01.08.2019
(51) Int. Cl.: C08L 83/07, C08K 3/36, C08K 5/56, C08L 83/05

(54) **LIQUID SILICONE RUBBER COMPOSITION, CURED OBJECT OBTAINED THEREFROM, ARTICLE INCLUDING SAID CURED OBJECT, AND METHOD FOR PRODUCING SILICONE RUBBER**

(30) Priority: 02.08.2018 JP 2018145835
(71) Applicant: Momentive Performance Materials Japan LLC, Tokyo 107-6119 (JP)
(72) Inventor: SHIMAKAWA, Masanari, Tokyo 107-6119 (JP)
(74) Representative: Gille Hrabal Partnerschaftsgesellschaft mbB Patentanwälte
(86) International application number: PCT/JP2019/030376
(87) International publication number: WO 2020/027302

(57) **Abstract**

There is provided silicone rubber that achieves both a low modulus and high tear strength and a liquid silicone rubber composition that gives the silicone rubber. The liquid silicone rubber composition contains: (A) an alkenyl group-containing linear polyorganosiloxane; (B) a 0.1 to 39 parts by mass linear hydrogenorganopolysiloxane relative to 100 parts by mass of (A); (C) a polyorganosiloxane having alkenyl groups and Si-H whose total number in one molecule is three or more; (D) a hydrosilylation catalyst; and (E) a silica powder having a specific surface area of 100 to 420 m²/g, the content of the silica powder being 10 to 50 parts by mass relative to 100 parts by mass of (A). Relative to the total mass of (A), (B), and (C), the total molar amount of the alkenyl groups of (A) and Si-H of (B) is 0.03 to 0.19 mmol/g, and the total molar amount of the alkenyl groups and SiH of (C) is 0.01 to 0.05 mmol/g. An Si-H/alkenyl group ratio of the composition is 0.7 to 1.3.

## Description

### FIELD

The present invention relates to a liquid silicone rubber composition, a cured product thereof, an article including the cured product, and a method for producing silicone rubber.

### BACKGROUND

Silicone rubber has excellent heat resistance, cold resistance, weather resistance, and flexibility owing to its molecular structure. In particular, addition-curable silicone rubber produced through the curing of an addition-curable polyorganosiloxane composition that contains a polyorganohydrogensiloxane having hydrogen atoms bonded to silicon atoms (Si-H), a polyorganosiloxane having alkenyl groups such as vinyl groups bonded to silicon atoms, and a hydrosilylation catalyst and that gives a cured product through an addition reaction of the hydrogen atoms bonded to the silicon atoms with the alkenyl groups is widely used in medical and health care applications requiring safety and as sealing materials of electronic devices and the like in which the mixture of impurities is not desired, because it is free of cracked residues of organic peroxide used as a vulcanizing agent, does not produce a reaction by-product through a condensation reaction as well as having heat resistance, cold resistance, weather resistance, and flexibility.

However, since the physical strength of silicone rubber in which polymers are simply crosslinked may be insufficient, a reinforcing material such as a silica powder is sometimes compounded to a silicone rubber composition to impart strength such as tensile strength, elongation, and tear strength suitable for the application where it is used. Silicone rubber compositions can be classified into two types, namely, a liquid silicone rubber composition and a solid millable silicone rubber composition according to the nature of a base polymer used and a compounding amount of the reinforcing material.

Having excellent fluidity, a liquid silicone rubber composition can be filled in a narrow space without a high pressure being applied in a forming method using a mold, making it possible to obtain a molded product having a complicated shape or a shape with a thin portion. Further, as compared with a millable silicone rubber composition for which an open device such as a twin roll is used when an additive such as a curing agent is compounded thereto, a liquid silicone rubber composition can be formed into a molded product using a closed device such as a pump or a static mixer and thus its use is preferred for producing a molded product used in medical applications and the like requiring a reduction of the risk of the mixture of foreign objects.

Further, reducing the viscosity of the liquid silicone rubber composition to improve its fluidity enables the adoption of a wide variety of forming methods such as coating, dipping, and screen printing in addition to injection molding and press forming without diluting it with a solvent or the like, making it possible to improve productivity and widen the application of silicone rubber.

To impart excellent fluidity to a liquid silicone rubber composition, it is a common practice to reduce a compounding amount of a reinforcing filler such as a silica powder as well as select a low-viscosity base polymer. This, however, tends to lower the physical strength such as tensile strength, elongation, tear strength, and so on of its cured product though improving the fluidity.

In recent years, in the applications of wound protection sheets, wearable devices, medical balloon catheters, and so on, in order to reduce stress to a worn part and facilitate releasing a molded product having a complicated and large undercut from a mold, there is a demand for silicone rubber that has both a low modulus and high tear strength. Further, in order to widen the degree of freedom of a forming method and the shape, there is a demand for an addition-curable silicone rubber composition that has a lower viscosity and excellent fluidity while maintaining a low modulus and high tear strength.

There have conventionally been proposed various addition silicone rubber compositions in which a linear polyorganosiloxane having, at its ends, hydrogen atoms bonded to silicon atoms is compounded as a chain extender for the purpose of obtaining low-viscosity and tough gel and elastomer cured product, improving molding durability in silicone rubber for molding, improving adhesiveness to a base material and stress relaxation properties, improving hardness, stretchability, tensile strength, and tear strength, and so on (see Patent References 1 to 4, for instance).

### RELEVANT REFERENCES

### PATENT REFERENCE

Patent References 1: JP-A Hei 07-188559
Patent References 2: JP-A 2004-231824
Patent References 3: JP-A 2002-1179921
Patent References 4: JP-A 2015-052026

### SUMMARY

### PROBLEM TO BE SOLVED BY THE INVENTION

The conventional silicone rubber compositions, however, have a difficulty in sufficiently achieving all of a low modulus, high tear strength, and excellent fluidity, and this is a cause of limiting the wider application of silicone rubber.

### MEANS FOR SOLVING THE PROBLEMS

The present invention was made to solve the aforesaid problem and has an object to provide a liquid silicone rubber composition excellent in fluidity that gives silicone rubber achieving both a low modulus and high tear strength, a cured product thereof, an article including the cured product, and a method for producing silicone rubber achieving both a low modulus and high tear strength.

A liquid silicone rubber composition of the present invention is a liquid silicone rubber composition containing:
(A) a linear polyorganosiloxane having two or more and less than three alkenyl groups in one molecule and not having a hydrogen atom bonded to a silicon atom;
(B) a linear polyorganosiloxane having two or more and less than three hydrogen atoms bonded to silicon atoms in one molecule and not having an alkenyl group, the content of the linear polyorganosiloxane being 0.1 to 39 parts by mass relative to 100 parts by mass of the component (A);
(C) a polyorganosiloxane having one kind or more of an alkenyl group and a hydrogen atom bonded to a silicon atom, a total number of the alkenyl groups and the hydrogen atoms bonded to the silicon atoms in one molecule being three or more;
(D) a hydrosilylation catalyst; and
(E) a silica powder having a specific surface area of 100 to 420 m²/g, the content of the silica powder being 10 to 50 parts by mass relative to 100 parts by mass of the component (A),
wherein a total molar amount of the alkenyl groups contained in the component (A) and the hydrogen atoms bonded to the silicon atoms contained in the component (B) relative to a total mass of the component (A), the component (B), and the component (C) is within a range of 0.03 to 0.19 mmol/g, a total molar amount of the alkenyl groups contained in the component (C) and the hydrogen atoms bonded to the silicon atoms, contained in the component (C) relative to the total mass of the component (A), the component (B), and the component (C) is within a range of 0.01 to 0.05 mmol/g, and a ratio of the number of the hydrogen atoms bonded to the silicon atoms to the number of the alkenyl groups, which are contained in the liquid silicone rubber composition, is within a range of 0.7 to 1.3.

In the liquid silicone rubber composition of the present invention, the component (A) preferably has a viscosity of 0.5 to 200 Pa·s.

In the liquid silicone rubber composition of the present invention, a molar amount of the alkenyl groups per mass in the component (A) is preferably 0.02 to 0.1 mmol/g.

In the liquid silicone rubber composition of the present invention, a total molar amount of the alkenyl groups and the hydrogen atoms bonded to the silicon atoms per mass in the component (C) is preferably 0.5 to 15 mmol/g.

The liquid silicone rubber composition of the present invention preferably has a viscosity of 5 to 2000 Pa s at 20°C.

The liquid silicone rubber composition of the present invention is a liquid silicone rubber composition whose cured product preferably has a modulus 300% of 0.2 to 1.7 MPa and a tear strength of 20 to 60 N/mm. Further, the cured product of the composition preferably has a fracture elongation of 800% or more.

The present invention provides silicone rubber that is a cured product of the liquid silicone rubber composition described above.

A method for producing silicone rubber of the present invention includes: mixing the following components (A) to (E) to obtain the liquid silicone rubber composition; and curing the liquid silicone rubber composition:
(A) a linear polyorganosiloxane having two or more and less than three alkenyl groups in one molecule and not having a hydrogen atom bonded to a silicon atom;
(B) a linear polyorganosiloxane having two or more and less than three hydrogen atoms bonded to silicon atoms in one molecule and not having an alkenyl group, the content of the linear polyorganosiloxane being 0.1 to 39 parts by mass relative to 100 parts by mass of the component (A);
(C) a polyorganosiloxane having one kind or more of an alkenyl group and a hydrogen atom bonded to a silicon atom, a total number of the alkenyl groups and the hydrogen atoms bonded to the silicon atoms in one molecule being three or more;
(D) a hydrosilylation catalyst; and
(E) a silica powder having a specific surface area of 100 to 420 m²/g, the content of the silica powder being 10 to 50 parts by mass relative to 100 parts by mass of the component (A),
wherein a total molar amount of the alkenyl groups contained in the component (A) and the hydrogen atoms bonded to the silicon atoms contained in the component (B) relative to a total mass of the component (A), the component (B), and the component (C) is within a range of 0.03 to 0.19 mmol/g,
a total molar amount of the alkenyl groups and the hydrogen atoms bonded to the silicon atoms, which are contained in the component (C) relative to a total mass of the component (A), the component (B), and the component (C) is within a range of 0.01 to 0.05 mmol/g, and
a ratio of the number of the hydrogen atoms bonded to the silicon atoms to the number of the alkenyl groups, which are contained in the liquid silicone rubber composition, is within a range of 0.7 to 1.3.

An article of the present invention is an article used as a wound protection sheet, a wearable device, or a medical balloon catheter and includes a cured product of the above-described liquid silicone rubber composition of the present invention.

### EFFECTS OF THE INVENTION

According to the present invention, it is possible to provide a liquid silicone rubber composition that gives silicone rubber achieving a low modulus, high tear strength, and excellent fluidity, a cured product thereof, an article including the cured product, and a method for producing silicone rubber that achieves both a low modulus and high tear strength.

### DETAILED DESCRIPTION

The liquid silicone rubber composition of the present invention contains the following components (A) to (E):
(A) a linear polyorganosiloxane having two or more and less than three alkenyl groups in one molecule and not having a hydrogen atom bonded to a silicon atom (component (A));
(B) a linear polyorganosiloxane having two or more and less than three hydrogen atoms bonded to silicon atoms in one molecule and not having an alkenyl group (component (B));
(C) a polyorganosiloxane having one kind or more of an alkenyl group and a hydrogen atom bonded to a silicon atom, a total number of the alkenyl groups and the hydrogen atoms bonded to the silicon atoms in one molecule being three or more (component (C));
(D) a hydrosilylation catalyst (component (D)); and
(E) a silica powder whose specific surface area is 100 to 420 m²/g (component (E))

In the liquid silicone rubber composition, the content of the component (E) is 10 to 50 parts by mass relative to 100 parts by mass of the component (A). Further, the content of the component (B) is 0.1 to 39 parts by mass to 100 parts by mass of the component (A).

In the liquid silicone rubber composition of the present invention, a total molar amount of the alkenyl groups contained in the component (A) and the hydrogen atoms bonded to the silicon atoms contained in the component (B) is within a range of 0.03 to 0.19 mmol/g relative to a total mass of the component (A), the component (B), and the component (C). A total molar amount of the alkenyl groups and the hydrogen atoms bonded to the silicon atoms, which are contained in the component (C), is within a range of 0.01 to 0.05 mmol/g relative to the total mass of the component (A), the component (B), and the component (C). A ratio of the number of the hydrogen atoms bonded to the silicon atoms to the number of the alkenyl groups, which are contained in the liquid silicone rubber composition, is within a range of 0.7 to 1.3.

The components contained in the liquid silicone rubber composition of the present invention will be hereafter described.

### <Component (A)>

The component (A), that is, the linear polyorganosiloxane having two or more and less than three alkenyl groups in one molecule and not having a hydrogen atom bonded to a silicon atom (hereinafter, also referred to as "(A) the alkenyl group-containing linear polyorganosiloxane) is a base polymer of the liquid silicone rubber composition of the present invention.

(A) the alkenyl group-containing linear polyorganosiloxane has two or more and less than three alkenyl groups in one molecule and does not have a hydrogen atom bonded to a silicon atom. (A) the alkenyl group-containing linear polyorganosiloxane preferably has the alkenyl groups only at its ends, and preferably does not have a reactive group such as an alkoxy group and a hydroxy group except the alkenyl groups. In the alkenyl group-containing linear polyorganosiloxane, a group or an atom bonded to the silicon atom, except the two or more and less than three alkenyl groups in one molecule, is preferably a substituted or unsubstituted monovalent hydrocarbon group not including an aliphatic unsaturated bond (hereinafter, also referred to as "the silicon atom-bonded hydrocarbon group") and is preferably composed only of the silicon atom-bonded hydrocarbon group.

One kind of (A) the alkenyl group-containing linear polyorganosiloxane may be used alone or two kinds or more thereof may be used in combination. If two kinds or more of (A) the alkenyl group-containing linear polyorganosiloxanes are used in combination, the number of the alkenyl groups in one molecule calculated in each kind may be within the aforesaid range. The liquid silicone rubber composition of the present invention may further contain another alkenyl group-containing linear polyorganosiloxane in which the number of the alkenyl groups in one molecule is different from and the other structure is the same as those of (A) the alkenyl group-containing linear polyorganosiloxane, as long as the effect of the present invention is not impaired.

The alkenyl group that (A) the alkenyl group-containing linear polyorganosiloxane has is typically an alkenyl group having two to six carbon atoms, preferably two to four carbon atoms, and more preferably two to three carbon atoms. Specific examples thereof include a vinyl group, an allyl group, a propenyl group, an isopropenyl group, a butenyl group, and an isobutenyl group, among which a vinyl group is preferable. The two or more and less than three alkenyl groups that (A) the alkenyl group-containing linear polyorganosiloxane has in one molecule may be the same or may be different.

The number of the alkenyl groups that (A) the alkenyl group-containing linear polyorganosiloxane has in one molecule is two or more and less than three. An especially preferable form of (A) the alkenyl group-containing linear polyorganosiloxane is such that one alkenyl group is bonded to each of its ends. If the number of the alkenyl groups is less than two, a rubber-like cured product is not obtained, and if it is three or more, it is not possible to achieve a low modulus and high tear strength.

To obtain a cured product achieving both a low modulus and high tear strength, the molar amount of the alkenyl groups that (A) the alkenyl group-containing linear polyorganosiloxane has is preferably 0.02 to 0.1 mmol/g relative to the total mass of the component (A).

The group or atom bonded to the silicon atom, other than the alkenyl groups, that (A) the alkenyl group-containing linear polyorganosiloxane has is preferably the silicon atom-bonded hydrocarbon group as described above, and the number of carbon atoms thereof is typically one to ten and preferably one to six.

Specific examples of the silicon atom-bonded hydrocarbon group include: alkyl groups such as a methyl group, an ethyl group, a propyl group, an isopropyl, a butyl group, an isobutyl group, a tert-butyl group, a pentyl group, a hexyl group, a cyclohexyl group, an octyl group, and a decyl group; aryl groups such as a phenyl group and a tolyl group; aralkyl groups such as a benzyl group and a phenylethyl group; and groups in which part or all of hydrogen atoms of these groups are replaced with halogen atoms such as chlorine, bromine, or fluorine, for example, a chloromethyl group and a 3,3,3-trifluoropropyl group. Among them, an alkyl group, an aryl group, and a 3,3,3-trifluoropropyl group are preferable because they have excellent properties of a low modulus and high tear strength, and a methyl group, a phenyl group, and a 3,3,3-trifluoropropyl group are more preferable. If (A) the alkenyl group-containing linear polyorganosiloxane has two or more silicon atom-bonded hydrocarbon groups in one molecule, they may be the same or may be different.

(A) the alkenyl group-containing linear polyorganosiloxane is substantially linear, but may have a few branches in part of its structure. In this case, in one molecule of (A) the alkenyl group-containing linear polyorganosiloxane, the total amount of a SiO₃/₂ unit and a SiO_{4/2} unit relative to the total of a SiO_{1/2} unit, a SiO_{2/2} unit, the SiO_{3/2} unit, and the SiO_{4/2} unit is preferably 5% or less in terms of molar percentage.

The viscosity of (A) the alkenyl group-containing linear polyorganosiloxane at 20 °C is preferably 0.5 to 200 Pa·s, and more preferably 1 to 100 Pa·s. By setting the viscosity of (A) the alkenyl group-containing linear polyorganosiloxane to 0.5 to 200 Pa·s, it is possible to be excellent in workability and to obtain a cured product having a low modulus and high tear strength. Note that, in this specification, the viscosity is a value measured with a rheometer under the condition of a 10 s⁻¹ shear rate and 20°C unless otherwise mentioned. As the rheometer, RS6000 manufactured by HAAKE is usable, for instance.

### <Component (B)>

(B) the linear polyorganosiloxane having two or more and less than three hydrogen atoms bonded to silicon atoms in one molecule (hereinafter, also referred to as "(B) the linear organohydrogenpolysiloxane ") acts as a chain extender.

(B) the linear organohydrogenpolysiloxane has two or more and less than three hydrogen atoms bonded to silicon atoms (hereinafter, also referred to as "the silicon atom-bonded hydrogen atoms") and does not have an alkenyl group. (B) the linear organohydrogenpolysiloxane preferably has the silicon atom-bonded hydrogen atoms only at its ends and does not preferably have a reactive group such as an alkoky group and a hydroxy group except the silicon atom-bonded hydrogen atoms. In (B) the linear organohydrogenpolysiloxane, a group or an atom bonded to the silicon atom, except the two or more and less than three silicon atom-bonded hydrogen atoms in one molecule, is preferably a silicon atom-bonded hydrocarbon group and is more preferably composed of only the silicon atom-bonded hydrocarbon group.

One kind of (B) the linear organohydrogenpolysiloxane may be used alone or two kinds or more thereof may be used in combination. If two kinds or more of (B) the linear organohydrogenpolysiloxanes are used in combination, the number of the silicon atom-bonded hydrogen atoms in one molecule calculated in each kind may be within the aforesaid range. The liquid silicone rubber composition of the present invention may further contain another linear organohydrogenpolysiloxane in which the number of the silicon atom-bonded hydrogen atoms in one molecule is different from and the other structure is the same as those of (B) the linear organohydrogenpolysiloxane, as long as the effect of the present invention is not impaired.

The number of the silicon atom-bonded hydrogen atoms that (B) the linear organohydrogenpolysiloxane has in one molecule is preferably two. An especially preferable form of (B) the linear organohydrogenpolysiloxane is such that one silicon atom-bonded hydrogen atom is bonded to each of its ends. If the number of the silicon atom-bonded hydrogen atoms is less than two, a rubber-like cured product is not obtained, and if it is three or more, it is not possible to achieve a low modulus and high tear strength.

The group or the atom bonded to the silicon atom except the silicon atom-bonded hydrogen atoms that (B) the linear organohydrogenpolysiloxane has is preferably the silicon atom-bonded hydrocarbon group as described above. Specific examples of the silicon atom-bonded hydrocarbon group that (B) the linear organohydrogenpolysiloxane has include the same groups as those exemplified for the silicon atom-bonded hydrocarbon group that (A) the alkenyl group-containing linear polyorganosiloxane has, and its preferable form is also the same. If (B) the linear organohydrogenpolysiloxane has two or more silicon atom-bonded hydrocarbon groups in one molecule, these may be the same or may be different.

(B) the linear organohydrogenpolysiloxane is substantially linear but may have a few branches in part of its structure. In this case, in one molecule of (B) the linear organohydrogenpolysiloxane, the total amount of a SiO_{3/2} unit and a SiO_{4/2} unit relative to the total of a SiO_{1/2} unit, a SiO_{2/2} unit, the SiO_{3/2} unit, and the SiO_{4/2} unit is preferably 5% or less in terms of molar percentage.

The viscosity of (B) the linear organohydrogenpolysiloxane is preferably 0.005 to 1 Pa·s, and more preferably 0.01 to 0.5 Pa·s. By setting the viscosity of (B) the linear organohydrogenpolysiloxane to 0.005 to 1 Pa·s, it is possible to be excellent in workability and to obtain a cured product having a low modulus and excellent properties.

An amount of the component (B) in the liquid silicone rubber composition of the present invention is 0.1 to 39 parts by mass relative to 100 parts by mass of the component (A). The amount of the component (B) is preferably 0.1 to 10 parts by mass. If the amount of the component (B) falls out of the aforesaid range, it is not possible to obtain a low modulus nor it is possible to obtain sufficient tear strength.

### <Component (C)>

The component (C) is a polyorganosiloxane that has one kind or more of an alkenyl group and a hydrogen atom bonded to a silicon atom (silicon atom-bonded hydrogen atom) and in which the total number of the alkenyl groups and the silicon atom-bonded hydrogen atoms in one molecule is three or more (hereinafter, also referred to as "(C) the crosslinkable polyorganosiloxane"). The component (C) acts as a crosslinking agent in the liquid silicone rubber composition of the present invention. Hereinafter, the alkenyl group and the silicon atom-bonded hydrogen atom will be also referred to as "the crosslinkable group".

(C) the crosslinkable polyorganosiloxane may be a polyorganosiloxane having, as the crosslinkable group, only three or more alkenyl groups in one molecule, or may be a polyorganosiloxane having, as the crosslinkable group, only three or more silicon atom-bonded hydrogen atoms in one molecule, or may be a polyorganosiloxane having, as the crosslinkable group, the alkenyl groups and the silicon atom-bonded hydrogen atoms in the total number of three or more in one molecule.

The number of the crosslinkable groups in one molecule in (C) the crosslinkable polyorganosiloxane is preferably 100 or less, and more preferably 50 or less from a viewpoint of keeping tear strength high.

A specific example of the component (C) is a polyorganosiloxane made up of three or more units selected from a unit represented by R¹₃SiO₁₂, a unit represented by R¹₂SiO_{2/2}, a unit represented by R¹SiO_{3/2} (where R¹ represents a silicon atom-bonded hydrocarbon group), a unit represented by SiO_{4/2}, a unit represented by R²₃SiO_{1/2}, a unit represented by R²₂SiO_{2/2} (where R² represents a hydrogen atom or a silicon atom-bonded hydrocarbon group, and one R² or more in one unit are the hydrogen atoms), a unit represented by R³₃SiO_{1/2}, and a unit represented by R³₂SiO_{2/2} (where R³ represents an alkenyl group or a silicon atom-bonded hydrogen group, and one R³ or more in one unit are the alkenyl groups), and includes, in one molecule, three or more units selected from the unit represented by R²₃SiO_{1/2}, the unit represented by R³₃SiO_{1/2}, the unit represented by R²₂SiO_{2/2}, and the unit represented by R³₂SiO_{2/2}.

Hereinafter, the unit represented by R¹₃SiO_{1/2} will be also referred to as R¹₃SiO_{1/2}. The same applies to the other units.

In the aforesaid units, if the plurality of R¹s are present in one molecule, they may be the same or may be different. Further, if the plurality of R²s are present in one molecule other than the hydrogen atom and the plurality of R³s are present in one molecule other than the alkenyl group, they may be the same or may be different.

The silicon atom-bonded hydrocarbon groups as R¹, R², and R³ are the same as the silicon atom-bonded hydrocarbon groups in the above-described component (A) and component (B), and their preferable forms are also the same, and they are each preferably a methyl group, a phenyl group, or a 3, 3,3-trifluoropropyl group because these have excellent properties of a low modulus and high tear strength.

The alkenyl group as R³ is the same as the alkenyl group in the above-described component (A), that is, it is an alkenyl group having two to six carbon atoms, preferably two to four carbon atoms, and more preferably two to three carbon atoms. Specific examples thereof include a vinyl group, an allyl group, a propenyl group, an isopropenyl group, a butenyl group, and an isobutenyl group, among which a vinyl group is preferable. The plurality of alkenyl groups contained in one molecule may be the same or may be different.

(C) the crosslinkable polyorganosiloxane is made up of three units or more selected from the aforesaid units (note that it includes three units or more selected from the R²₃SiO_{1/2} unit, the R³₃SiO_{1/2} unit, the R²₂SiO_{2/2} unit, and the R³₂SiO_{2/2} unit), and the total number of the units is preferably not less than three nor more than 1000.

(C) the crosslinkable polyorganosiloxane is preferably (C) a crosslinkable polyorganosiloxane in which all of the three or more crosslinkable groups in one molecule are the alkenyl groups (hereinafter, (Ca) the crosslinkable polyorganosiloxane) or (C) a crosslinkable polyorganosiloxane in which all of the three or more crosslinkable groups in one molecule are the silicon atom-bonded hydrogen atoms (hereinafter, (Cb) the crosslinkable polyorganosiloxane).

The molecular structure of (C) the crosslinkable polyorganosiloxane may be either lineared or branched. Further, one kind of (C) the crosslinkable polyorganosiloxane may be used alone, or two kinds or more thereof may be used in combination. If two kinds or more of (C) the crosslinkable polyorganosiloxanes are used in combination, the number of the alkenyl groups and the silicon atom-bonded hydrogen atoms and the number of the aforesaid units calculated in each kind may be within the aforesaid ranges.

If two kinds or more of (C) the crosslinkable polyorganosiloxanes are used in combination as the component (C), one kind of (Ca) the crosslinkable polyorganosiloxane or more and one kind of (Cb) the crosslinkable polyorganosiloxane or more are preferably combined.

In the component (C), one kind of (C) the used crosslinkable polyorganosiloxane by itself is preferably liquid at room temperature and preferably has a viscosity falling within a range of 0.0001 to 100 Pa·s. The liquid silicone rubber composition of the present invention may contain a crosslinkable polyorganosiloxane in which the number of the alkenyl groups and the silicon atom-bonded hydrogen atoms and the number of the aforesaid units are different and the other structure is the same, as long as the effects of the present invention are not impaired.

In the component (C), the molar amount of the crosslinkable groups, that is, the total molar amount of the alkenyl groups and the silicon atom-bonded hydrogen atoms is preferably 0.5 to 15 mmol/g relative to the total mass of the component (C). Further, in each of (C) the crosslinkable polyorganosiloxanes forming the component (C) as well, the molar amount of the aforesaid crosslinkable groups, that is, the total molar amount of the alkenyl groups and the silicon atom-bonded hydrogen atoms is preferably 0.5 to 15 mmol/g. This makes it possible to easily obtain a cured product that achieves both a low modulus and high tear strength.

In the liquid silicone rubber composition of the present invention, the total molar amount of the alkenyl groups contained in the component (A) and the silicon atom-bonded hydrogen atoms contained in the component (B) is within a range of 0.03 to 0.19 mmol/g relative to the total mass of the component (A), the component (B), and the component (C). If the total molar amount of the alkenyl groups contained in the component (A) and the silicon atom-bonded hydrogen atoms contained in the component (B) is less than 0.03 mmol/g, the liquid silicone rubber composition becomes high in viscosity to lose fluidity. If the total molar amount is over 0.19 mmol/g, sufficient crosslinking is not possible and excellent tear strength cannot be obtained. The total molar amount of the alkenyl groups contained in the component (A) and the silicon atom-bonded hydrogen atoms contained in the component (B) is preferably 0.032 to 0.18 mmol/g, and more preferably 0.032 to 0.161 mmol/g.

In the liquid silicone rubber composition of the present invention, the total molar amount of the alkenyl groups and the silicon atom-bonded hydrogen atoms (crosslinkable groups) contained in the component (C) is within a range of 0.01 to 0.05 mmol/g relative to the total mass of the component (A), the component (B), and the component (C). If the total molar amount of the crosslinkable groups contained in the component (C) is less than 0.01 mmol/g, a sufficient crosslink density cannot be obtained, and thus a rubber-like cured product cannot be obtained. If the total molar amount of the crosslinkable groups is over 0.05 mmol/g, the modulus of the cured product becomes too high and its tear strength is not sufficient. The total molar amount of the crosslinkable groups contained in the component (C) relative to the total mass of the component (A), the component (B), and the component (C) is more preferably 0.01 to 0.045 mmol/g, and more preferably 0.01 to 0.035 mmol/g.

In the component (A), the component (B), and the component (C), the molar amount of the alkenyl groups and the molar amount of the silicon atom-bonded hydrogen atoms can be measured using ¹H-NMR.

In the liquid silicone rubber composition of the present invention, a ratio of the number of the silicon atom-bonded hydrogen atoms to the number of the alkenyl groups, that is, a molar ratio of the silicon atom-bonded hydrogen atoms to the alkenyl groups which are calculated from their total numbers in all the components, is within a range of 0.7 to 1.3. If the aforesaid ratio of the number of the silicon atom-bonded hydrogen atoms to the number of the alkenyl groups falls out of the aforesaid range, sufficient tear strength cannot be obtained. The aforesaid ratio of the number of the silicon atom-bonded hydrogen atoms to the number of the alkenyl groups is more preferably 0.8 to 1.24, and still more preferably 0.9 to 1.10. Usually, the aforesaid ratio of the number of the silicon atom-bonded hydrogen atoms to the number of the alkenyl groups is a ratio of the total number of the silicon atom-bonded hydrogen atoms to the total number of the alkenyl groups contained in the component (A), the component (B), and the component (C).

### <(D) hydrosilylation catalyst>

In the liquid silicone rubber composition of the present invention, (D) the hydrosilylation catalyst is a catalyst for promoting an addition reaction of the alkenyl groups contained in the component (A) and the component (C) and the silicon atom-bonded hydrogen atoms contained in the component (B) and the component (C). The component (D) is selected from a group consisting of metals Pt, Pd, Rh, Co, Ni, Ir, and Ru and metal compounds of these and preferably contains platinum or at least one kind of a platinum compound. The platinum compound as the component (D) can be selected from a group consisting of an organic platinum compound and a salt of platinum, for instance. The platinum compound as the component (D) may have a solid carrier such as activated carbon, carbon, or a silica powder.

The organic platinum compound is suitably a photoactivatable catalyst containing a (η-diolefin)-(σ-aryl)-platinum complex, a η⁵ cyclopentadienyl platinum complex compound, and a complex having a σ-bonded ligand, preferably a cyclopentadienyl ligand that is optionally replaced with a σ-bonded alkyl or allyl ligand. Another example of the photoactivatable platinum catalyst that can be used is one having a ligand selected from diketones.

The platinum compound may be in any form of platinum(0), (II), and (VI) compounds capable of forming a complex with phosphite ester.

Examples of the platinum compound include a Pt(0)-alkenyl complex whose ligand is alkenyl or alkenyl such as cycloalkenyl and a Pt(0)-alkenyl siloxane complex whose ligand is alkenyl siloxane such as vinyl siloxane. A Pt(0) complex with 1,3-divinyltetramethyldisiloxane or 2,4,6,8-tetravinyl-2,4,6,8-tetramethyltetrasiloxane is especially preferable because of its good dispersibility to the polyorganosiloxane composition.

A compounding amount of the component (D) may be an effective amount and can be appropriately increased/decreased according to a desired curing speed. If a platinum-based catalyst is used as the component (D), its compounding amount relative to the total mass of the component (A), the component (B), and the component (C) is typically, for example, within a range of 0.1 to 1,000 ppm and preferably 0.1 to 300 ppm in terms of the mass of platinum atoms. Increasing this compounding amount causes no change in curing properties and is not economical.

### <(E) silica powder>

In the liquid silicone rubber composition of the present invention, the silica powder as the component (E) acts as a reinforcing material of silicone rubber produced through the curing of the liquid silicone rubber composition. The silica powder as the component (E) has a specific surface area of 100 to 420 m²/g and preferably 130 to 300 m²/g. If the specific surface area is less than 100 m²/g, the tear strength of the silicone rubber becomes insufficient, and if it is over 420 m²/g, the viscosity increases to lower workability. The specific surface area is a value measured by a BET method.

As (E) the silica powder, fine-powder silica is usable, and a synthetic silica powder such as a dry silica powder of fumed silica or a wet silica powder is used, for instance. Having a large amount of a silanol group on their surfaces, these silica powders each can be used as what is called a surface-treated silica powder that is surface-treated in advance with a surface treatment agent such as, for example, halogenated silane, alkoxysilane, or any of various kinds of silazane compounds.

Further, at the time when the silica powder as the component (E) is compounded to the alkenyl group-containing linear polyorganosiloxane as the component (A), by compounding the halogenated silane, the alkoxysilane, or any of the silazane compounds, water, and so on and kneading the resultant by a known method, and subsequently removing an excessive amount of the surface treatment agent and a by-product due to a reaction by heating or pressure reduction, it is possible to treat the surface of the silica powder during the kneading process.

An amount of (E) the silica powder in the liquid silicone rubber composition of the present invention is 10 to 50 parts by mass relative to 100 parts by mass of the component (A). The composition in which the amount of (E) the silica powder is over 50 parts by mass relative to 100 parts by mass of the component (A) has poor workability, and the composition in which this amount is less than 10 parts by mass does not have sufficient tear strength. In the case where the silica powder that is surface-treated in advance is used, the amount before the surface treatment may be calculated as the amount of the silica powder since the surface treatment causes little mass change of the silica powder.

### <Other optional components>

In the composition of the present invention, optional components besides the aforesaid components (A) to (E) can be compounded within a range not impairing the object of the present invention. Examples of the optional components include a polyorganosiloxane not containing a reactive group such as a silicon atom-bonded hydrogen atom and an alkenyl group, a reaction inhibitor, a heat resistance imparting agent, a flame retardant, a thixotropic additive, a pigment, a dye, a conductivity imparting agent, a thermal conductivity imparting agent, and a dielectricity imparting agent.

### [Liquid Silicone Rubber Composition]

It is possible to prepare the liquid silicone rubber composition of the present invention by mixing the above-described components (A) to (E) (including the optional components if the optional components are compounded) by a common method. At this time, the components to be mixed may be one part or may be divided into two parts or more as required when they are mixed. For example, in the case where they are divided into two parts, they can be divided into a part composed of part of the components (A) and the components (B), (C), (E) and a part composed of the rest of the component (A) and the component (D) at the time of the mixing.

The components obtained in the above-described manner can also be divided into a first part composed of part of the component (A) and the surface-treated silica powder as the component (E), a second part composed of part of the component (A), the component (B), and the component (C), and a third part composed of the rest of the component (A) and the component (D) at the time of the mixing. In this case, it is preferable to mix the third part after the first part and the second part are mixed.

The liquid silicone rubber composition of the present invention is a liquid composition, that is, exhibits fluidity at room temperature. Because the liquid silicone rubber composition of the present invention is a liquid composition, any of various molding methods can be selected. The viscosity at 20 °C of the liquid silicone rubber composition of the present invention before it cures is preferably 5 to 2000 Pa·s, and more preferably 5 to 500 Pa·s. The viscosity of the liquid silicone rubber composition before the curing is regarded as its viscosity before (D) the hydrosilylation catalyst (it may be compounded with a slight amount of the component (A)) is compounded after the components (A) to (C) and (E) are mixed.

### [Method for Producing Silicone Rubber]

A method for producing silicone rubber of the present invention includes: mixing the aforesaid components (A) to (E) to obtain the liquid silicone rubber composition of the present invention (mixing step); and curing the liquid silicone rubber composition (curing step).

The mixing step can be executed by the same method as that described above in the preparation of the liquid silicone rubber composition of the present invention.

The curing step is a step of curing the liquid silicone rubber composition of the present invention at room temperature or under a temperature condition according to its use to obtain silicone rubber. The curing temperature is, for example, 20 to 200°C, and the curing time is, for example, 0.001 to 24 hours. Further, in the case where the photoactivatable catalyst is used as the component (D), by activating the catalyst by light irradiation and then causing the curing at room temperature or by heating, it is possible to obtain the silicone rubber as a cured product of the liquid silicone rubber composition.

As a result of curing the liquid silicone rubber composition of the present invention, the silicone rubber having a modulus 300% of 0.2 to 1.7 MPa and a tear strength of 20 to 60 N/mm can be obtained as the cured product. The modulus 300% of the silicone rubber is more preferably 0.2 to 1.0 MPa, and its tear strength is more preferably 30 to 60 N/mm. Further, this silicone rubber achieves a fracture elongation of 800% or more. Note that the modulus 300% and the fracture elongation are values measured according to DIN 53 504 S2. The tear strength is a value measured according to ASTM D624 dieB.

The silicone rubber of the present invention which is the cured product of the liquid silicone rubber composition of the present invention is suitable for use where its flexibility and high strength in addition to its heat resistance, cold resistance, weather resistance, safety, and so on are made use of. For example, an article including the silicone rubber of the present invention is suitable for medical applications such as wound protection sheets and medical balloon catheters and applications where it comes into contact with skin, such as wearable devices. In particular, its use for forming a mold of a molded product having a large undercut brings about a great effect.

### EXAMPLES

Next, examples will be described. The present invention is not limited to the following examples.

Components used in the examples and comparative examples and abbreviations in the description of the components are as follows.

### <Abbreviations>

M: unit represented by (CH₃)₃SiO_{1/2}
M^{Vi}: unit represented by (CH₂=CH)(CH₃)₂SiO_{1/2}
M^{H}: unit represented by H(CH₃)₂SiO_{1/2}
D: unit represented by (CH₃)₂SiO_{2/2}
D^{Vi}: unit represented by (CH₂=CH)(CH₃)SiO_{2/2}
D^{H}: unit represented by H(CH₃)SiO_{2/2}
T^{Ph}: unit represented by (C₆H₅)SiO_{3/2}
Q: unit represented by SiO_{4/2}
Vi: vinyl group
SiH: silicon atom-bonded hydrogen atom

### < Components used>

(A) an alkenyl group-containing linear polyorganosiloxane
   (A1) M^{Vi}D₃₃₅M^{Vi} (vinyl group content: 0.08 mmol/g, viscosity 3 Pa·s)
   (A2) M^{Vi}D₉₂₉M^{Vi} (vinyl group content: 0.029 mmol/g, viscosity 80 Pa·s)
   (A3) M^{Vi}D₃₈₅₈M^{Vi} (vinyl group content: 0.007 mmol/g, viscosity 7700 Pa·s)
   (A4) M^{Vi}D₁₄₈M^{Vi} (vinyl group content: 0.18 mmol/g, viscosity 0.4 Pa·s)
(B) a linear organohydrogenpolysiloxane
   M^{H}D₂₂M^{H} (silicon atom-bonded hydrogen atom content: 1.13 mmol/g, viscosity: 0.019 Pa·s)
(C) a crosslinkable polyorganosiloxane
   (C1) D^{Vi}₄ (vinyl group content: 11.6 mmol/g)
   (C2) M^{Vi}D₄₂₆D^{Vi}₂₈M^{Vi} (vinyl group content: 0.88 mmol/g, viscosity 6.8 Pa·s)
   (C3) M^{H}₃T^{Ph} (silicon atom-bonded hydrogen atom content: 9.09 mmol/g)
   (C4): MD^{H}₂₀D₁₈M (silicon atom-bonded hydrogen atom content: 7.5 mmol/g, viscosity 0.029 Pa s)
   (C5): M^{H}₈Q₄ (silicon atom-bonded hydrogen atom content: 10.3 mmol/g, viscosity 0.021 Pa·s)
(D) a hydrosilylation catalyst
   A 1,3-divinyl-1,1,3,3-tetramethyldisiloxane solution containing a 1,3-divinyl-1,1,3,3-tetramethyldisiloxane platinum(0) complex as the component (D) whose platinum-equivalent content is 20% by mass is compounded at a 0.1% concentration to the component (A1).
(E) a silica powder
   (E1) Aerosil 200 (manufactured by EVONIK, BET specific surface area 200 m²/g)
   (E2) Aerosil 300 (manufactured by EVONIK, BET specific surface area 300 m²/g)

### (Preparation of Base Compounds (BC))

The component (A), the silica powder as the component (E), 1,1,1,3,3,3-hexamethyldisilazane (HMDZ), and water were mixed at the ratios shown in Table 1 and the mixtures were kneaded at a temperature of 50°C or lower for thirty minutes using a planetary mixer. Thereafter, kneading by heating was performed at 150°C for one hour, and kneading by heating under reduced pressure was further performed at a temperature of 150°C for one hour to remove an excessive amount of HMDZ, the water, and a volatile product, whereby base compounds (BC1 to BC5) were prepared. In the preparation of the base compounds, the silica powder as the component (E) was surface-treated with HMDZ, whereby (E') a surface-treated silica powder was prepared.

As for BC4, since a raw rubber base polymer was used, the component (A), the silica powder as the component (E), HMDZ, and the water were mixed at the ratio shown in Table 1 and the mixture was kneaded at a temperature of 50°C or lower for thirty minutes using a kneader. Thereafter, the kneading by heating was performed at 150°C for three hours while a nitrogen gas was made to flow, to remove an excessive amount of HMDZ, the water, and the volatile product, whereby the base compound was prepared.

**[Table 1]**

| | | BC1 | BC2 | BC3 | BC4 | BC5 |
|---|---|---|---|---|---|---|
| (A) (Part by mass) | (A1) M^{Vi}DₘM^{Vi} | 800 | | 1000 | | |
| | (A2) M^{Vi}D₉₂₉M^{Vi} | | 800 | | 400 | |
| | (A3) M^{Vi}D₃₈₅₈M^{Vi} | | | | 400 | |
| | (A4) M^{Vi}D₁₄₈M^{Vi} | | | | | 1000 |
| (E) (Part by mass) | (E1) AEROSIL 200 | 400 | 400 | | 400 | 400 |
| | (E2) AEROSIL 300 | | | 400 | | |
| Surface treatment agent | HMDZ | 80 | 80 | 100 | 80 | 80 |
| | Water | 50 | 50 | 50 | 50 | 50 |
| Viscosity of BC (Pas) | | 297 | 1486 | 1740 | not liquid⁽¹⁾ | 164 |

| | | | | | | |
|---|---|---|---|---|---|---|
| (1) not liquid: The compound did not have fluidity like millable silicone. | | | | | | |

Using the base compounds obtained in the above, the component (A), a chain extender as the component (B), and a crosslinking agent as the component (C) were mixed such that the compositions corresponding to the types and amounts became as shown in Second part in Table 2, followed by stirring. The viscosity of each of the obtained mixtures was measured, and thereafter the component (A1) containing the hydrosilylation catalyst as the component (D) was added as a third part in the amounts shown in Table 2, followed by stirring, whereby liquid silicone rubber compositions (Examples 1 to 11) were prepared. The viscosity was measured with a rheometer (manufactured by HAAKE, RS6000) under the condition of a 10 s⁻¹ shear rate and 20 °C according to DIN 53 018. Similarly, liquid or millable silicone rubber compositions (Comparative examples 1 to 4) having the compositions shown in Table 3 were prepared.

Note that the parenthesized numerical values shown in the (D) section in Tables 2, 3 indicate mass ppm in terms of platinum atoms (Pt) relative to the total mass of the component (A), the component (B), and the component (C).

The silicone rubber compositions obtained above were degassed under reduced pressure and pressed at 150°C for ten minutes using a mold with a 2 mm thickness, whereby rubber sheets were fabricated. Thereafter, the rubber sheets were taken out from the mold and post-cured in a 200°C oven for four hours, whereby sheets for physical property measurement were obtained.

Regarding the obtained sheets, hardness, density, tensile strength, fracture elongation, modulus 300%, and tear strength were measured in conformity with the following prescriptions. The evaluation results of these are shown in Tables 2, 3 together with the compositions of the examples.

### <Physical Property Measurement Method>

Hardness (Shore A hardness): Three 2 mm sheets were stacked and the hardness was measured with a shore A durometer according to DIN 53 505.

Specific gravity: it was measured according to DIN 53 479A.

Tensile strength, fracture elongation and modulus 300%: They were measured according to DIN 53 504 S2.

Tear strength: It was measured according to ASTM D624 dieB.

Further, in the examples, the total molar amount of alkenyl groups contained in the component (A) and hydrogen atoms bonded to silicon atoms contained in the component (B) relative to the total mass of the components (A), (B), and (C) (in Tables, "Vi+SiH in (A) and (B)"), a molar amount of alkenyl groups and hydrogen atoms bonded to silicon atoms, which were contained in the component (C) (in Tables, "Vi or SiH of (C)"), a ratio of the hydrogen atoms bonded to the silicon atoms to the number of (A) the alkenyl groups, which are contained in the component (A), the component (B), and the component (C) (in Tables, "SiH/Vi ratio"), and the total amount of the components (A), (B), and (C) (in Tables, "total of A+B+C",) were calculated, and they are shown in Tables 2, 3 in the lower sections of Composition.

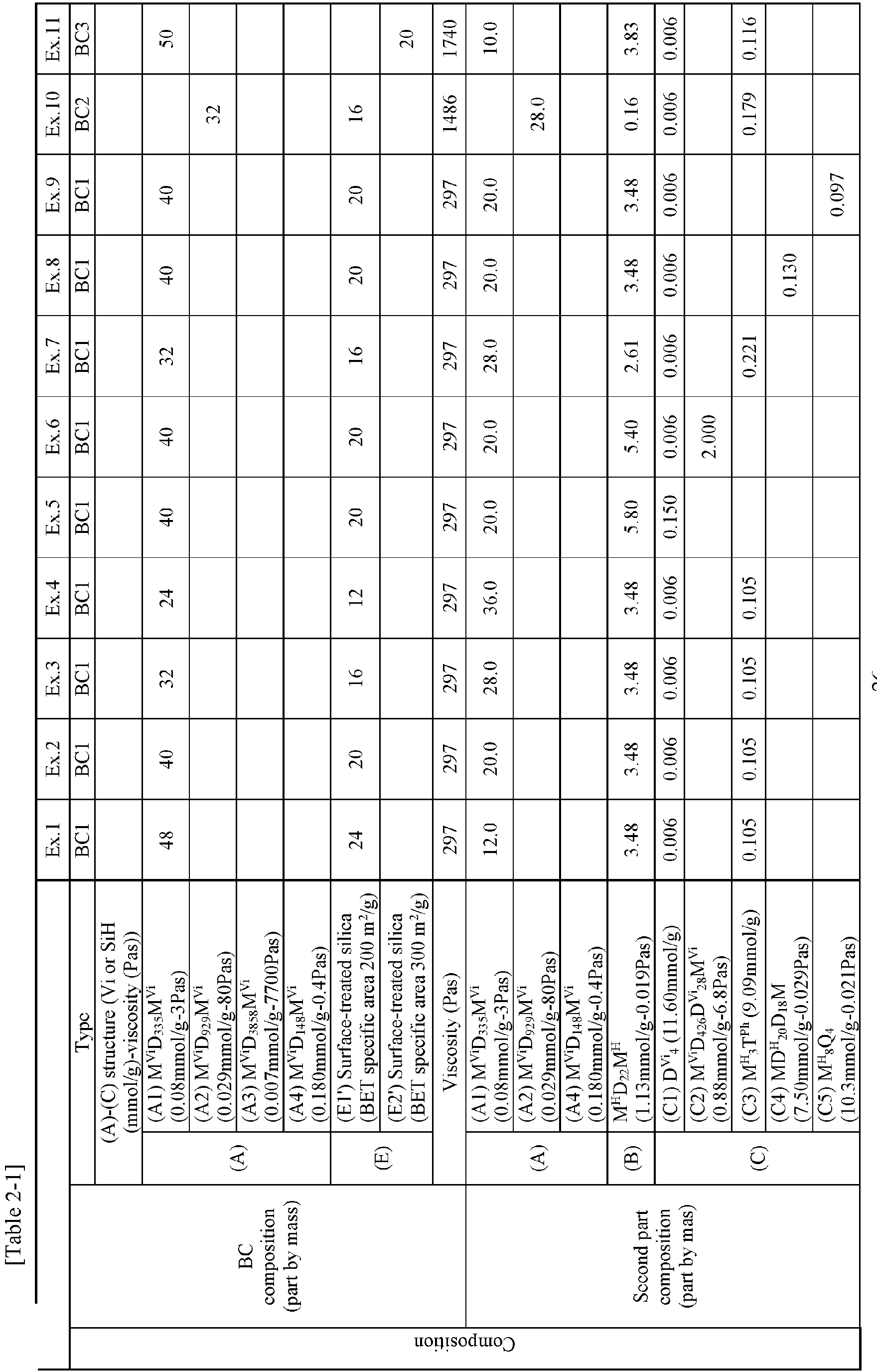

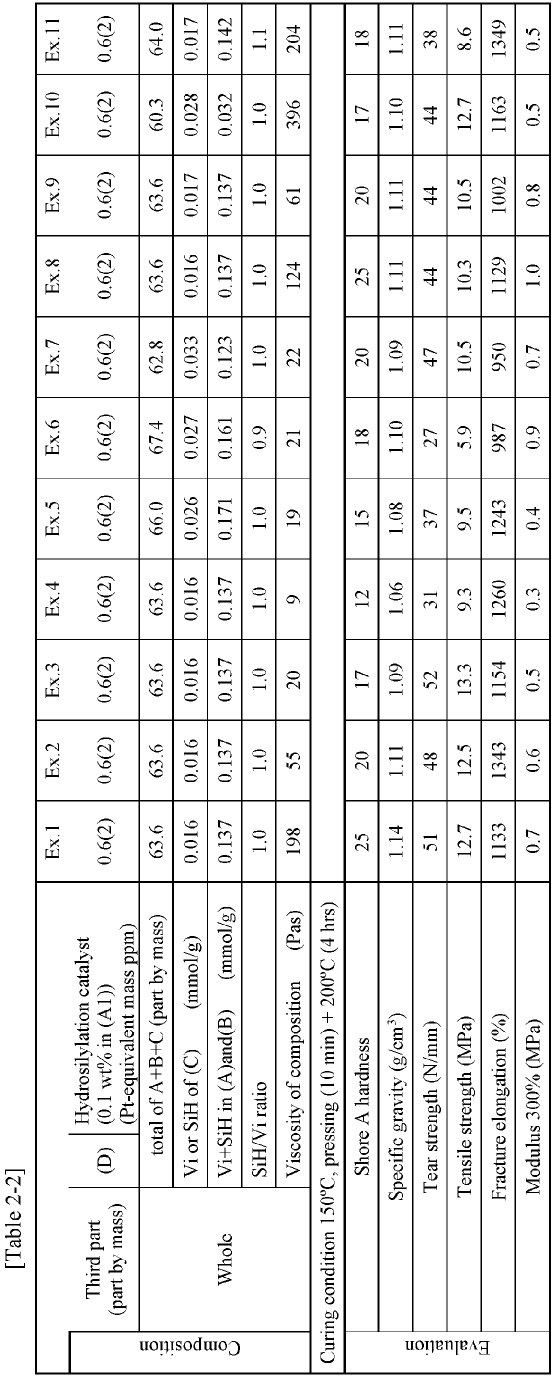

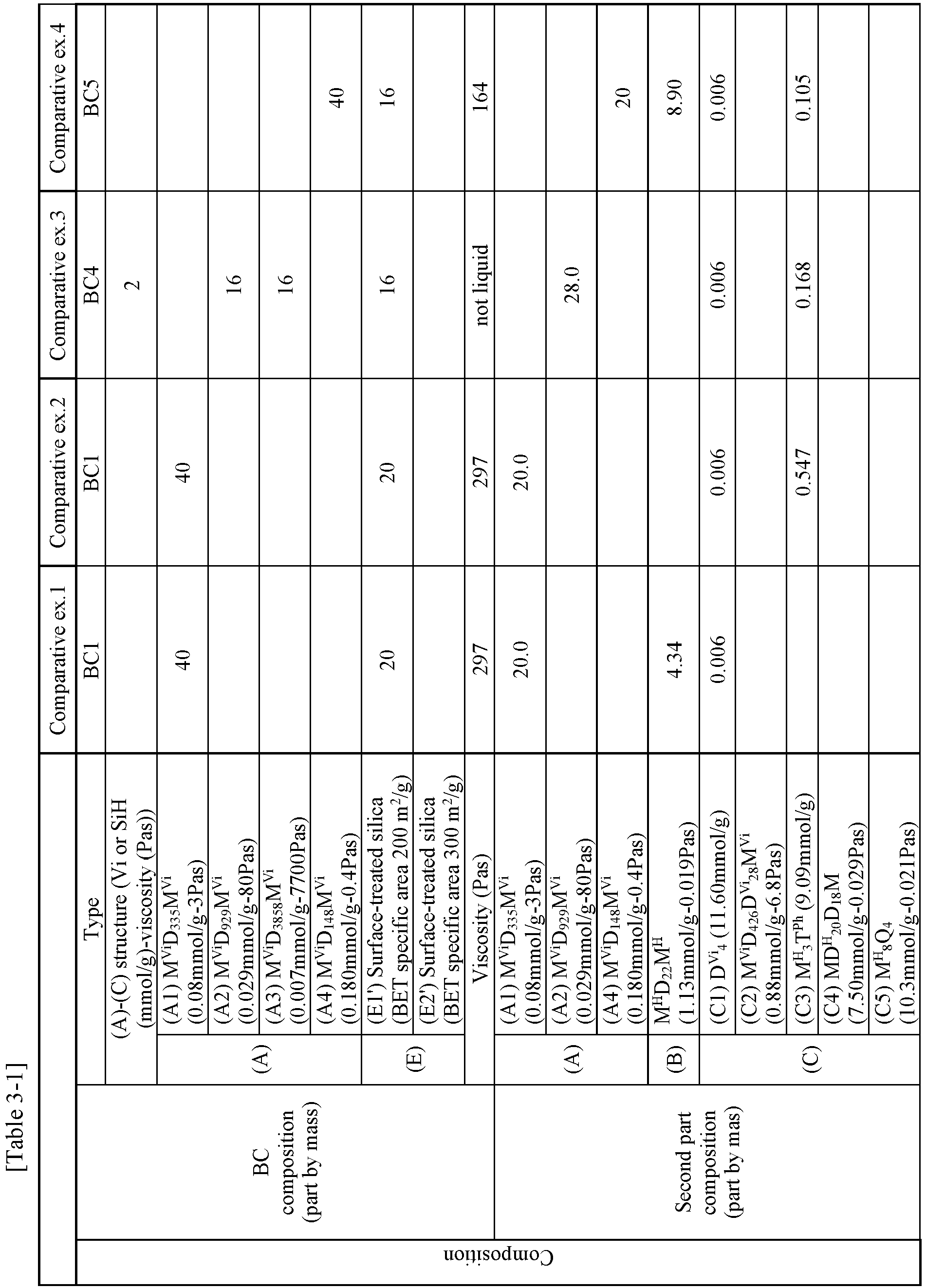

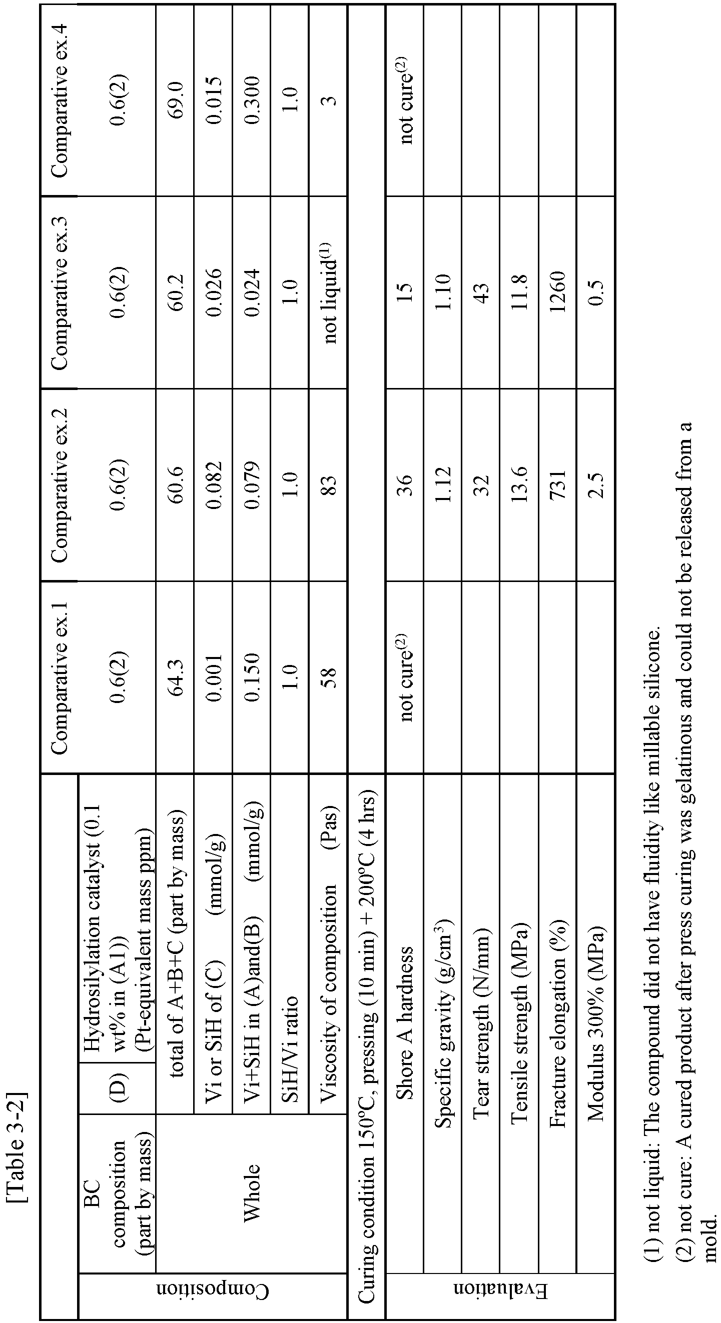

While certain embodiments of the present invention have been described above, these embodiments have been presented by way of example only, and are not intended to limit the scope of the invention. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A liquid silicone rubber composition containing:
(A) a linear polyorganosiloxane having two or more and less than three alkenyl groups in one molecule and not having a hydrogen atom bonded to a silicon atom;
(B) a linear polyorganosiloxane having two or more and less than three hydrogen atoms bonded to silicon atoms in one molecule and not having an alkenyl group, the content of the linear polyorganosiloxane being 0.1 to 39 parts by mass relative to 100 parts by mass of the component (A);
(C) a polyorganosiloxane having one kind or more of an alkenyl group and a hydrogen atom bonded to a silicon atom, a total number of the alkenyl groups and the hydrogen atoms bonded to the silicon atoms in one molecule being three or more;
(D) a hydrosilylation catalyst; and
(E) a silica powder having a specific surface area of 100 to 420 m²/g, the content of the silica powder being 10 to 50 parts by mass relative to 100 parts by mass of the component (A),
wherein a total molar amount of the alkenyl groups contained in the component (A) and the hydrogen atoms bonded to the silicon atoms contained in the component (B) relative to a total mass of the component (A), the component (B), and the component (C) is within a range of 0.03 to 0.19 mmol/g,
a total molar amount of the alkenyl groups contained in the component (C) and the hydrogen atoms bonded to the silicon atoms contained in the component (C) relative to the total mass of the component (A), the component (B), and the component (C) is within a range of 0.01 to 0.05 mmol/g, and
a ratio of the number of the hydrogen atoms bonded to the silicon atoms to the number of the alkenyl groups, which are contained in the liquid silicone rubber composition, is within a range of 0.7 to 1.3.

2. The liquid silicone rubber composition according to claim 1, wherein the component (A) has a viscosity of 0.5 to 200 Pa·s.

3. The liquid silicone rubber composition according to claim 1 or 2, wherein a molar amount of the alkenyl groups per mass in the component (A) is 0.02 to 0.1 mmol/g.

4. The liquid silicone rubber composition according to any one of claims 1 to 3, wherein a total molar amount of the alkenyl groups and the hydrogen atoms bonded to the silicon atoms per mass in the component (C) is 0.5 to 15 mmol/g.

5. The liquid silicone rubber composition according to any one of claims 1 to 4, the liquid silicone rubber composition having a viscosity of 5 to 2000 Pa·s at 20°C.

6. The liquid silicone rubber composition according to any one of claims 1 to 5, a cured product of the liquid silicone rubber composition having a modulus 300% of 0.2 to 1.7 MPa and a tear strength of 20 to 60 N/mm.

7. The liquid silicone rubber composition according to any one of claims 1 to 6, the cured product of the liquid silicone rubber composition having a fracture elongation of 800% or more.

8. A cured product of the liquid silicone rubber composition according to any one of claims 1 to 7.

9. A method for producing silicone rubber, the method comprising:
mixing the following components (A) to (E) to obtain the liquid silicone rubber composition; and
curing the liquid silicone rubber composition:
(A) a linear polyorganosiloxane having two or more and less than three alkenyl groups in one molecule and not having a hydrogen atom bonded to a silicon atom;
(B) a linear polyorganosiloxane having two or more and less than three hydrogen atoms bonded to silicon atoms in one molecule and not having an alkenyl group, the content of the linear polyorganosiloxane being 0.1 to 39 parts by mass relative to 100 parts by mass of the component (A);
(C) a polyorganosiloxane having one kind or more of an alkenyl group and a hydrogen atom bonded to a silicon atom, a total number of the alkenyl groups and the hydrogen atoms bonded to the silicon atoms in one molecule being three or more;
(D) a hydrosilylation catalyst; and
(E) a silica powder having a specific surface area of 100 to 420 m²/g, the content of the silica powder being 10 to 50 parts by mass relative to 100 parts by mass of the component (A),
wherein a total molar amount of the alkenyl groups contained in the component (A) and the hydrogen atoms bonded to the silicon atoms contained in the component (B) relative to a total mass of the component (A), the component (B), and the component (C) is within a range of 0.03 to 0.19 mmol/g,
a total molar amount of the alkenyl groups contained in the component (C) and the hydrogen atoms bonded to the silicon atoms contained in the component (C) relative to the total mass of the component (A), the component (B), and the component (C) is within a range of 0.01 to 0.05 mmol/g, and
a ratio of the number of the hydrogen atoms bonded to the silicon atoms to the number of the alkenyl groups, which are contained in the liquid silicone rubber composition, is within a range of 0.7 to 1.3.

10. An article comprising the cured product according to claim 8, the article being used as a wound protection sheet, a wearable device, or a medical balloon catheter.
